(19) **Europäisches Patentamt / European Patent Office / Office européen des brevets**

(11) **EP 2 658 374 B1**

(12) # EUROPEAN PATENT SPECIFICATION

(45) Date of publication and mention of the grant of the patent:
**25.11.2015 Bulletin 2015/48**

(51) Int Cl.:
***A61K 31/122*** (2006.01)     ***A01N 35/00*** (2006.01)
***A61P 1/16*** (2006.01)

(21) Application number: **11852682.1**

(22) Date of filing: **29.12.2011**

(86) International application number:
**PCT/US2011/067733**

(87) International publication number:
**WO 2012/092430 (05.07.2012 Gazette 2012/27)**

(54) **HEPATOPROTECTANT ACTIVITY OF GARCINOL**

LEBERSCHÜTZENDE WIRKUNG VON GARCINOL

ACTIVITÉ HÉPATOPROTECTRICE DU GARCINOL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2010 US 201061428643 P**

(43) Date of publication of application:
**06.11.2013 Bulletin 2013/45**

(73) Proprietor: **Majeed, Muhammed**
**East Windsor, NJ 08520-5321 (US)**

(72) Inventors:
• **BANI, Sarang**
  **Karnataka 560058 (IN)**
• **MAJEED, Muhammed**
  **East Windsor, NJ 08520-5321 (US)**

(74) Representative: **Ehnis, Tobias et al**
**Dr. Gassner & Partner mbB**
**Patentanwälte**
**Marie-Curie-Strasse 1**
**91052 Erlangen (DE)**

(56) References cited:
WO-A2-2010/025410    US-A1- 2002 187 943
US-A1- 2002 187 943    US-A1- 2007 010 483
US-A1- 2010 062 090

• CHENG, A. ET AL.: "Garcinol inhibits cell growth in hepatocellular carcinoma Hep3B cells through induction of ROS-dependent apotosis", FOOD FUNCT, vol. 1, 27 October 2010 (2010-10-27), pages 301-307, XP002723079,
• Padhye, S. et al.: "Emerging role of garcinol, the antioxidant chalcone from Garcinia indica Chisy and its synthetic analogs", Journal of Hematology & Oncology, vol. 2, no. 38 2 September 2009 (2009-09-02), XP002723080, Retrieved from the Internet: URL:http://www.ncbi.nlm.nih.gov/pmc/articl es/PMC2743703/pdf/1756-8722-2-38.pdf [retrieved on 2014-04-09]
• MORON M.S. ET AL.: 'LEVELS OF GLUTATHIONE, GLUTATHIONE REDUCTASE AND GLUTATHIONE S-TRANSFERASE ACTIVITIES IN RAT LUNG AND LIVER' BIOCHIMICA ET BIOPHYSICA ACTA vol. 582, 1979, pages 67 - 78, XP025789351
• MCCLAIN ET AL.: 'Cytokines and Alcoholic Liver Disease' ALCOHOL HEALTH & RESEARCH WORLD vol. 21, no. 4, 1997, pages 317 - 320, XP055086308
• FARHOOD A. ET AL.: 'Intercellular adhesion molecule 1 (ICAM-1) expression and its role in neutrophil- induced ischemia-reperfusin injury in rat liver.' JOURNAL OF LEUKOCYTE BIOLOGY vol. 57, March 1995, pages 368 - 374, XP055086311
• SANDERSON N. ET AL.: 'Hepatic expression of mature transforming growth factor (81 in transgenic mice results in multiple tissue lesions.' PROC. NATL. ACAD. SCI. USA vol. 92, March 1995, pages 2572 - 2576, XP055086313

**Description**

This application is a non-provisional application of provisional application 61/428643 filed on December 30, 2010.

**FIELD OF THE INVENTION**

The invention in general relates to medicaments for hepatotoxicity (hepatic toxicity) management. More specifically, it relates to the hepatoprotective potential of garcinol.

**DESCRIPTION OF PRIOR ART**

The term "hepatotoxicity" refers in general to the chemical induced liver damage. Such damage occurs mainly when the liver discharges its innate function of transformation and clearance of chemicals in the body.

The close association of the liver to the gastrointestinal tract and the spleen through the portal venous system, as part of its metabolic function further exacerbates the impact of toxicity due to circulating drugs.

Nilesh Mehta et al (Drug-Induced Hepatotoxicity- Medscape REFERENCE-Drugs, Diseases and Procedures) reports that 75% of the idiosyncratic drug reactions lead to liver transplantation or death. The same authors also report that in the United States, approximately 2000 cases of acute liver failure occur annually and 50% of such cases could be attributed to the effects of drugs. The authors further elucidate the pathophysiological mechanisms underlying drug induced hepatotoxicity. These include,

(i) Hepatocyte disruption that follows actin fibril disassembly caused due to the covalent binding of the drug to the intracellular proteins and the decrease in ATP levels.
(ii) Drug induced blockage of transport pumps preventing normal bile excretion to cause cholestasis.
(iii) Immune response mediated by the binding of the drug to P-450 enzyme.
(iv) TNF-$\alpha$ mediated apoptosis of hepatocytes.
(v) Mitochondrial disruption due to decreased ATP production brought about by the drug induced inhibition of NAD and FAD in the beta-oxidation energy production mechanism.
(vi) Toxic metabolites induced bile duct injury.
(vii) Activation of liver cell types like the Browicz-Kuppfer cells and receptors like toll-like receptor 4 (TLR4) and CD14 thereof during early ethanol induced liver damage, leading to internalization of the lipopolysaccaride fraction of the cell walls of gram negative bacteria that flourish in the gut in an alcohol rich environment. This then leads to activation of pro-inflammatory cytokines like TNF-$\alpha$ and superoxides which would enter the stellate cells in the liver, leading to collagen synthesis, fibrosis and eventually liver cirrhosis.

Hepatoprotection as an ongoing therapeutic (both preventive and prophylactic) means, thus assumes tremendous importance in conditions where there is interplay of one or more of the aforesaid mechanisms in causing liver damage. Such conditions include,

(i) Nonalcoholic steatohepatitis (NASH) which represents fat in the liver and ensuing inflammation thereof. Though it resembles alcoholic liver disease, it occurs in people who are non-alcoholics or who consume very little alcohol. NASH is enigmatic in the sense that it may regress on its own or worsen in a slow manner leading to fibrosis and subsequently life threatening cirrhosis. Further, no specific therapies exist for this condition except life style management methods.
(ii) Alcohol abuse resulting from habitual or prolonged consumption;
(iii) Chemotherapy for cancer.
(iv) Conditions such as non-alcoholic fatty liver disease where there is fat deposition in the liver without signs and symptoms of inflammation or liver damage.
(v) Viral induced acute or chronic hepatitis, where the latter condition may lead to fibrosis and life threatening cirrhosis/liver cancer.

Garcinol, isolated from Garcinia sp. fruit rind is known in the art as an antioxidant and chemo protective agent. (Tanaka, T. et.al. Prevention of colonic aberrant crypt foci by dietary feeding of garcinol in male F3444 rats. Carcinogenesis, June 2000: 21 (6): 1183-9). From Cheng, A.-C. et al., Food Funct., 2010, 1, 301-307 it is known that garcinol inhibits cell growth in hepatocellular carcinoma Hep3B cells through induction of ROS-dependent apoptosis.

Garcinol and isogarcinol were evaluated for their antibacterial activity against methicillin-resistant Staphylococcus *aureus* (Linuma M et al, Antibacterial activity of some Garcinia benzophenone derivatives against methicillin-resistant Staphylococcus aureus. Biol Pharm Bull 1996 February; 19(2): 311-4).

Garcinol's role as a potent inhibitor of histone acetyltransferases (HATs) both *in vitro* and *invivo* was reported by Tapas et al in 2004 ("Polyisoprenylated Benzophenone, Garcinol, a Natural Histone Acetyl transferase Inhibitor, Represses Chromatin Transcription and Alters Global GeneExpression", The Journal of Biological Chemistry, Vol. 279, No. 32, Issue of August 6, pp.33716-33726, 2004).

The present inventors add further to the medical potential of garcinol in disclosing the molecule's hepatoprotectant activity through modulation of one or more pathophysiological effects highlighted herein above. In specific, the present inventors have sought to study the effects of garcinol in modulating the biochemical markers associated with hepatotoxicity.

## SUMMARY OF THE INVENTION

The present invention discloses the potential of garcinol as a hepatoprotectant. Protection of cultures of Hep-2 cells in the presence of effective concentrations of garcinol has been demonstrated. Also demonstrated is garcinol mediated modulation of biochemical markers in animal models of toxin-$CCl_4$ induced, drug-Paracetamol induced and alcohol induced hepatoxicity.

## BRIEF DESCRIPTION OF DRAWINGS

FIG 1: shows the graphical representation of the effect of different doses of garcinol on TGF-beta 1 expression (pg/ml) in the liver homogenates from toxin ($CCl_4$) induced hepatotoxic animal models. Values are expressed as Mean $\pm$ SE, n = 6); P value * : <0.001.

FIG 2: shows the graphical representation of the effect of different doses of garcinol on Inter-Cellular Adhesion Molecule 1 (ICAM-1 or CD52) expression (pg/ml) in the liver homogenates from toxin ($CCl_4$) induced hepatotoxic animal models. Values are expressed as Mean $\pm$ SE, n = 6); P value * : <0.001.

FIG 3: shows the graphical representation of the effect of different doses of garcinol on C-reactive protein expression (mg/ml) in the liver homogenates from toxin ($CCl_4$) induced hepatotoxic animal models. Values are expressed as Mean $\pm$ SE, n = 6); P value * : <0.001.

FIG 4: shows the graphical representation (flow cytometric studies) of the effect of different doses of garcinol on Tumor Necrosis Factor-$\alpha$ (TNF-$\alpha$) in the blood drawn from toxin ($CCl_4$) induced hepatotoxic animal models.

FIG 5: shows the graphical representation (flow cytometric studies) of the effect of different doses of garcinol on Interleukin-2 (IL-2) in the blood drawn from toxin ($CCl_4$) induced hepatotoxic animal models.

FIG 6: shows the graphical representation of the effect of different doses of garcinol on Interleukin-4 (IL-4) in the blood drawn from toxin ($CCl_4$) induced hepatotoxic animal models.

FIG 7: shows the graphical representation of the effect of different doses of garcinol on TGF-beta 1 expression (pg/ml) in the liver homogenates from drug (Paracetamol) induced hepatotoxic animal models. Values are expressed as Mean $\pm$ SE, n = 6); P value * : <0.01., **: <0.001., GC : Garcinol; APAP : Paracetamol.

FIG 8: shows the graphical representation of the effect of different doses of garcinol on Inter-Cellular Adhesion Molecule 1 (ICAM-1 or CD52) expression (pg/ml) in the liver homogenates from drug (Paracetamol) induced hepatotoxic animal models. Values are expressed as Mean $\pm$ SE, n = 6); P value * : <0.01., **: <0.001., GC : Garcinol; APAP : Paracetamol.

FIG 9: shows the graphical representation (flow cytometric studies) of the effect of different doses of garcinol on Tumor Necrosis Factor-$\alpha$ (TNF-$\alpha$) in the blood drawn from drug (Paracetamol) induced hepatotoxic animal models.

FIG 10: shows the graphical representation of the effect of different doses of garcinol on Tumor Necrosis Factor-$\alpha$ (TNF-$\alpha$) and Interleukin-1$\beta$ (IL-1$\beta$) in the serum of alcohol (Ethyl alcohol) induced hepatotoxic animal models. Values are expressed as Mean $\pm$ SE, n = 6); P value * : <0.01., **: <0.001., GC : Garcinol.

FIG 11: shows the graphical representation of the effect of different doses of garcinol on Interleukin-12 (IL-12) in the serum of alcohol (Ethyl alcohol) induced hepatotoxic animal models.

**FIG 12:** shows the graphical representation of the cytotoxic potential (% cytotoxicity) of garcinol in Hep G2 liver cancer cell line in comparison with Silymarin, a known hepatoprotective agent.

**FIG 13:** shows the graphical representation of the hepatoprotective effect of garcinol in comparision with the known hepatoprotective agent Silymarin in Hep G2 liver cancer cell line.

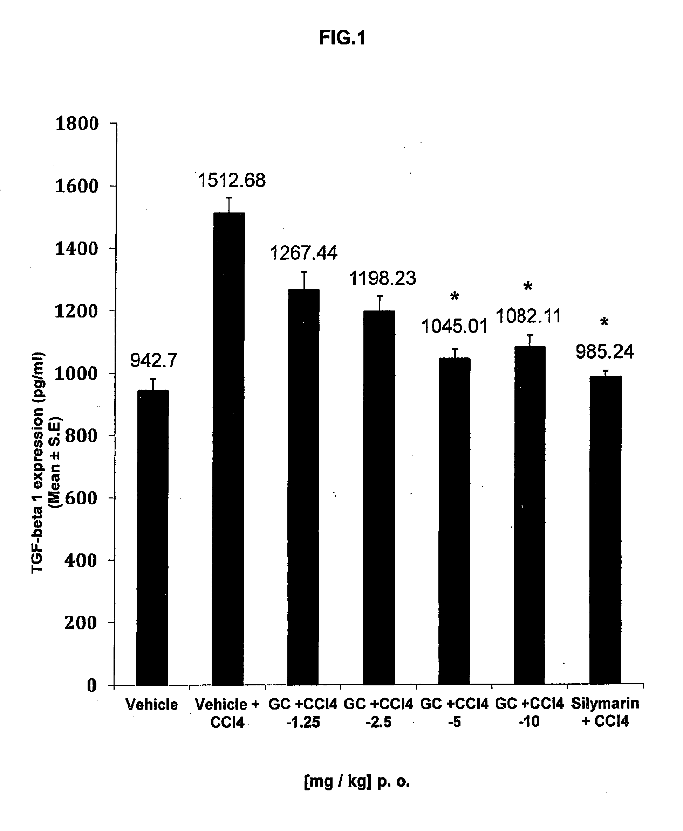

## DETAILED DESCRIPTION (FIGS 1-13)-PREFERRED EMBODIMENT

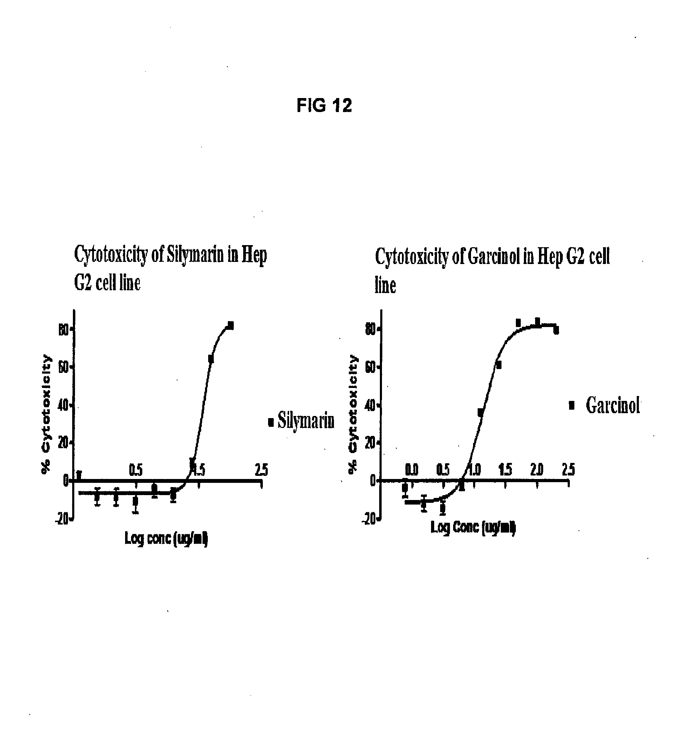

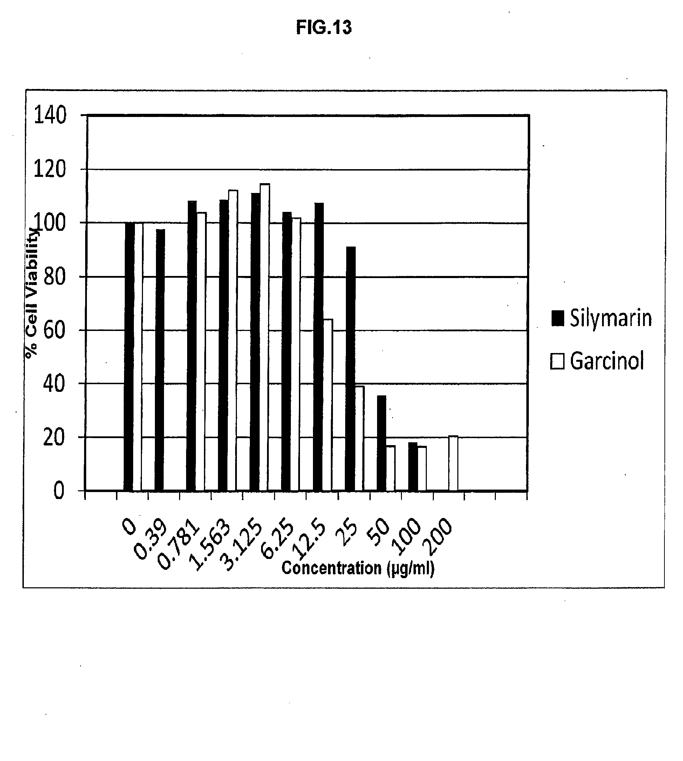

In the most preferred embodiment, the present invention pertains to a method of mammalian hepatocyte protection, said method comprising step of bringing into contact mammalian hepatocytes with an effective concentration of garcinol, wherein any method practiced as a method for treatment of the human or animal body by therapy or practiced as a diagnostic method on the human or animal body is excluded. More specifically, the effective concentration of garcinol is from about 0.78 $\mu$g/ml to about 6.25 $\mu$/ml (FIGS. 12 and 13). In an alternate most preferred embodiment, the present invention relates to garcinol for use as a medicament for providing hepatoprotection, more specifically, hepatoprotection for a mammal.

In another preferred embodiment, the present invention relates to a method of reducing increased levels of cytokine expression in mammalian models of liver damage (hepatotoxicity), said method comprising step of administering an effective amount of garcinol to said models **(FIGS. 1, 4, 5, 6, 7, 9, 10 and 11).** In specific embodiments, the cytokines are Transforming Growth Factor G-$\beta$1 (TGF- $\beta$1), Tumor Necrosis Factor-$\alpha$, Interleukin-2 (IL-2), Interleukin-4 (IL-4) and Interleukin-12 (IL-12). In further specific embodiment, hepatotoxicity in mammalian models may be induced by toxin, drug or ethyl alcohol. In an alternate specific embodiment, liver damage is induced by combinations of toxin, drug and ethyl alcohol.

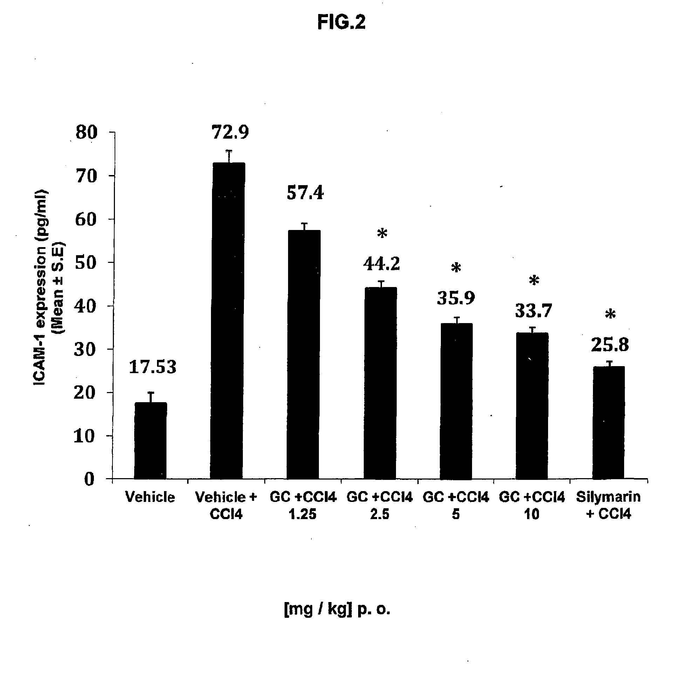

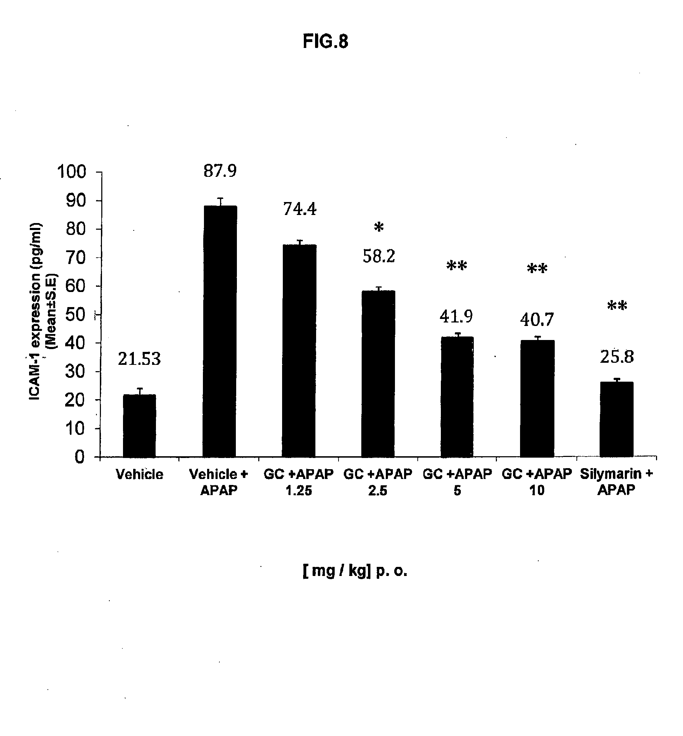

In yet another preferred embodiment, the present invention relates to a method of reducing increased levels of adhesion molecule expression in mammalian models of liver damage (hepatotoxicity) induced by toxins and/or drugs, said method comprising step of administering an effective amount of garcinol to said models **(FIGS.2 and 8).** In a specific embodiment, the adhesion molecule is Intracellular Adhesion Molecule-1 (ICAM-1 or CD 52).

In yet another preferred embodiment, the present invention relates to a method of reducing elevated levels of liver enzymes and/or bile pigments in mammalian models of liver damage (hepatotoxicity), said method comprising step of administering an effective amount of garcinol to said models. In specific embodiments, the liver enzymes are Alanine Transaminase, Aspartate aminotransferase and Alkaline phosphatase **(Tables 3, 4 and 5).** In another specific embodiment, the bile pigment is bilirubin. In further specific embodiment, liver damage in mammalian models may be induced by toxin, drug or ethyl alcohol. In an alternate specific embodiment, liver damage is induced by combinations of toxin, drug and ethyl alcohol.

The potential therapeutic value of garcinol as a hepatoprotectant may be understood through examples elucidated herein below.

## EXAMPLE 1

Acute Oral Safety of Garcinol: No mortality was observed up to 2000 mg/kg. p. o. in mice up to two weeks of observation. The parameters studied and observations recorded are included in **Table 1.** (OECD Guidelines for Testing of Chemicals. Guideline 423, Acute Oral Toxicity - Acute Toxic Class Method, Adopted, (1996). Organization for Economic Cooperation and Development).

**Table 1: Parameters studied on acute oral safety of garcinol**

| General Behavior | Dermal |
|---|---|
| Aggressiveness: Nil<br>Fearful: Nil<br>Passive: Nil<br>General Movement: Normal<br>General Locomotor Activity: Normal | Blanching: Nil<br>Hyperemia: Nil<br>Cyanosis: Nil |
| **Central Nervous System** | **General Observations** |
| Excitation: Nil<br>Motor Activity: Normal | Muscular Weakness: Nil<br>Salivation: Normal |

(continued)

| Central Nervous System | General Observations |
|---|---|
| Tremors: Nil | Pilo erection: Nil |
| Clonic convulsions: Nil | Diarrhea: Nil |
| **Respiratory System** | **Reflexes** |
| Respiration rate: Normal | Corneal: No Effect |
| Respiration Depth: Normal | Pinnal: No Effect |
| **Autonomic Nervous System** | **Food and Water (Intake/Excretion)** |
| Motor Activity: Normal | Fecal Output: Normal |
| Atexia: Nil | Urine Output: Normal |
| Respiration Rate: Normal | |
| Diarrhea: Nil | |

## EXAMPLE 2

**Effect of different doses of Garcinol (GC) on TGF-β1, ICAM-1 and CRP expressions in the liver homogenates of carbon tetrachloride induced liver damage in rats.**

Animals used in the experiment: Male Wistar Rats

Weight of the animals: 140-160 grams

Doses of Garcinol (GC) used for the study: 1.25, 2.5, 5, 10 mg/kg p.o.

Standard drug: Silymarin (50 mg/kg) p.o.

**Procedure:** Liver injury was induced by administration of carbon tetrachloride ($CCl_4$) mixed with liquid paraffin (5 fold dilution). Animals were given single dose of $CCl_4$ at 1ml/kg, p.o. followed by the administration of garcinol at different time intervals (Table 2). [(i) Bramanti G, Murmann W, Pierini P and Comporti M (1978). Effect of cicloxilic acid on CCl4 - induced liver injury. Drug Research 28: 1112-1217. (ii) B Singh, A K Saxena, B K Chandan, K K Anand (1998). Hepatoprotective activity of Verbenalin on experimental liver damage in rodents. Fitoterapia LXIX 2: 135-140. **(iii)** B.K. Chandan, A.K. Sharma, K.K. Anand Boerhaavia diffusa: A study of its hepatoprotective activity. Journal of Ethnopharmacology Volume 31, Issue 3, March 1991, Pages 299-307].

**Table 2: provides details of day and time schedule of $CCl_4$ and garcinol administration**

| Day and time Schedule of toxin/drug (GC) Administration | | Treatment | Time elapsed since toxin administration |
|---|---|---|---|
| Day 1 | 10 A.M. | $CCl_4$ | 0h |
| | 4 P.M. | GC | 6h after $CCl_4$ |
| Day 2 | 10 A.M. | GC | 24h after $CCl_4$ |
| Day 3 | 10 A.M. | GC | 48h after $CCl_4$ |
| | 12 Noon | Samples Collection (liver & blood) | 50h after $CCl_4$/2h after last treatment of test material (GC) |

Liver tissue was homogenized on ice with a polytron and homogenate was centrifuged at 5000 g for 15 min. Aliquots of the supernatant were separated and used for biochemical analysis. Supernatants were stored at -80 °C until cytokine analysis. TGFβ, ICAM-1, C-reactive protein (CRP) were estimated using commercially available kits based on sandwich and competitive ELISA technique according to the manufacturers' instructions. All cytokine concentrations were carried out by means of colorimetric measurement at 450 nm on an ELISA plate reader by interpolation from a standard curve. [(i) Magari K, Miyata S, Ohkubo Y, Mutoh S, (2004). Inflammatory cytokine levels in paw tissues during development of rat collagen-induced arthritis: Effect of FK506, an inhibitor of T cell activation. Inflammation Research. 53: 469-474 (Magari *et al.*, 2004); and (ii) Modulation of Thl/Th2 cytokines and inflammatory mediators by hydroxychavicol in adjuvant induced arthritic tissues. Anjali Pandey, Sarang Bani, Prabhu Dutt, Krishna Avtar Suri. Cytokine 49 (2010)114-121]

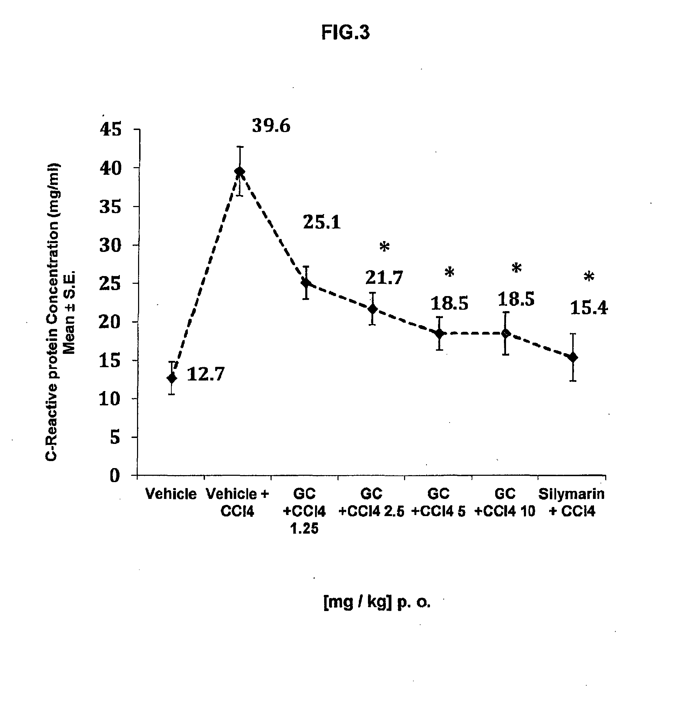

**Results :** Garcinol inhibited increased levels of TGFβ (FIG 1) ICAM-1 (FIG 2), C-reactive protein (CRP) (FIG 3) associated with acute hepatitis resulting from carbon tetrachloride induced liver damage in rats.

## EXAMPLE 3

**Effect of different doses of Garcinol (GC) on TNF-$\alpha$, IL-2 and IL-4 in the blood of carbon tetrachloride induced liver damage in rats.**

Animals used in the experiment: Male Wistar Rats

Weight of the animals: 140-160 grams

Doses of Garcinol (GC) used for the study: 1.25, 2.5, 5, 10 mg/kg p.o.

Standard drug: Silymarin (50 mg/kg) p.o.

**Procedure:** Liver injury was induced by administration of carbon tetrachloride ($CCl_4$) mixed with liquid paraffin (5 fold dilution). Animals were given single dose of $CCl_4$ at 1ml/kg, p.o. followed by the administration of garcinol (See aforesaid Table 2). [(i) Bramanti G, Murmann W, Pierini P and Comporti M (1978). Effect of cicloxilic acid on CCl4 - induced liver injury. Drug Research 28: 1112-1217. (ii) B Singh, A K Saxena, B K Chandan, K K Anand (1998). Hepatoprotective activity of Verbenalin on experimental liver damage in rodents. Fitoterapia LXIX 2: 135-140. (iii) B.K. Chandan, A.K. Sharma, K.K. Anand Boerhaavia diffusa: A study of its hepatoprotective activity. Journal of Ethnopharmacology, Volume 31, Issue 3, March 1991, Pages 299-307].

The animals were bled retro-orbitally and blood was collected in EDTA-coated tubes for the estimation of PE-labeled anti-rat TNF-alpha, IL-2 ad IL-4 monoclonal antibody expression. Analysis was done on flow cytometer (BD-FACS CANTO II). These fluorochrome-labeled monoclonal antibodies were added directly to 100 $\mu$l of whole blood, which was then lysed using whole blood lysing reagent. Following the final centrifugation, samples were resuspended in phosphate-buffered saline (pH, 7.4) and analyzed directly on the flow cytometer. A fluorescence trigger was set on the PE (FL2) parameter to collect the events. [(i) Bani S, Kaul A, Khan B, Ahmad SF, Suri KA, Gupta BD, Satti NK, Qazi GN, (2006).Suppression of T lymphocyte activity by lupeol isolated from Crataeva religiosa. Phytotherapy Research; 20(4): 279-287. And (ii) Bani S, Kaul A, Khan B, Ahmad SF, Suri KA, Satti NK, (2005). Immunosuppressive properties of an ethyl acetate fraction from Euphorbia royleana. Journal of Ethnopharmacology; 99: 185-192].

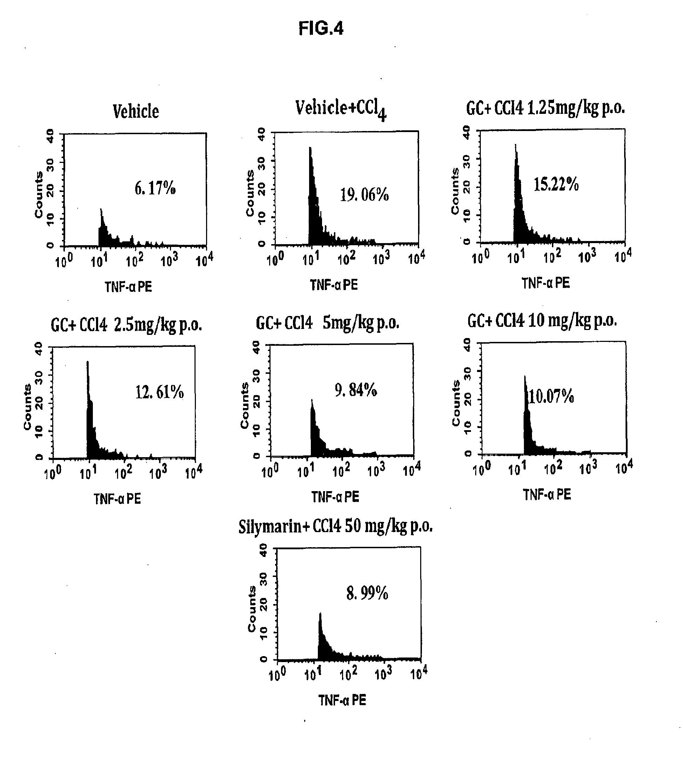

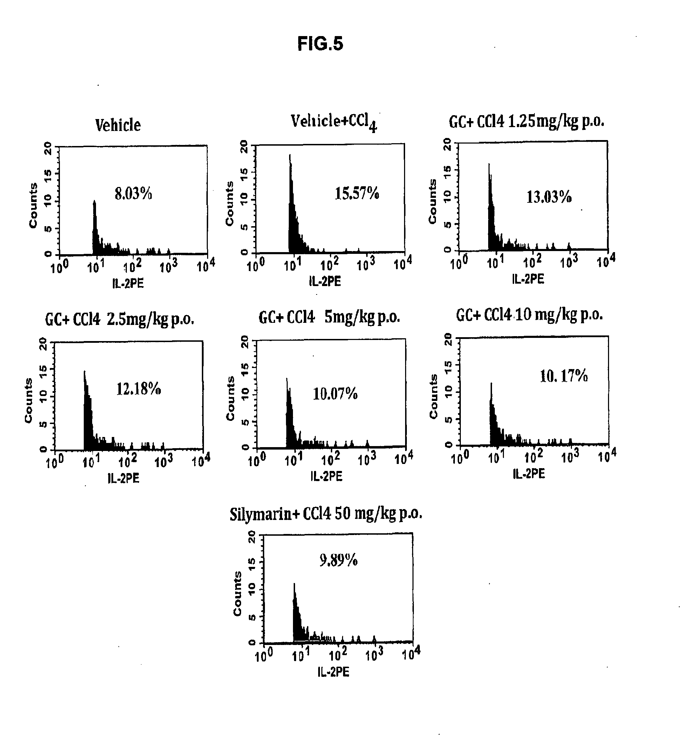

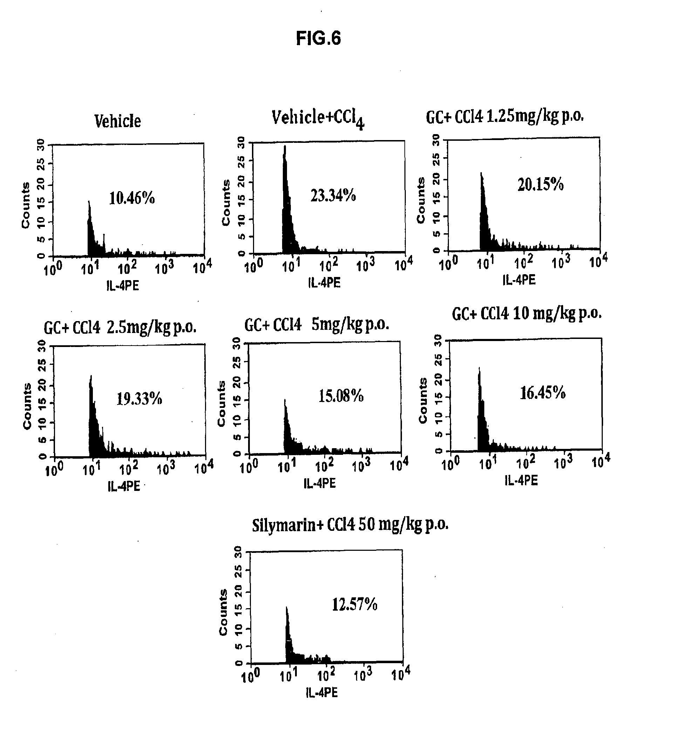

**Results:** Garcinol (GC) caused a dose based inhibition of T-Cell immune response marked by inhibition of TNF-$\alpha$ (FIG 4), IL-2 (FIG 5) and IL-4 (FIG 6) expressed in the blood of carbon tetrachloride induced liver damage in rats.

## EXAMPLE 4

**Effect of different doses of Garcinol (GC) on bilirubin and serum enzymes Alanine Transaminase (ALT), Aspartate Aminotransferase (AST) and Alkaline Phosphatase (ALP) in carbon tetrachloride induced liver damage in rats**

Animals used in the experiment: Male Wistar Rats

Weight of the animals: 140-160 grams

Doses of Garcinol (GC) used for the study: 1.25, 2.5, 5, 10 mg/kg p.o.

Standard drug: Silymarin (50 mg/kg) p.o.

**Procedure:** Liver injury was induced by administration of carbon tetrachloride ($CCl_4$) mixed with liquid paraffin (5 fold dilution). Animals were given single dose of $CCl_4$ at 1ml/kg, p.o. followed by the administration of garcinol (Table 2). [(i) Bramanti G, Murmann W, Pierini P and Comporti M (1978). Effect of cicloxilic acid on CCl4 - induced liver injury. Drug Research 28: 1112-1217. (ii) B Singh, A K Saxena, B K Chandan, K K Anand (1998). Hepatoprotective activity of Verbenalin on experimental liver damage in rodents. Fitoterapia LXIX 2: 135-140. (iii) B.K. Chandan, A.K. Sharma, K.K. Anand Boerhaavia diffusa: A study of its hepatoprotective activity. Journal of Ethnopharmacology, Volume 31, Issue 3, March 1991, Pages 299-307].

Blood was collected from the retro-orbital plexus of experimental animals and no anti-coagulant was added. Blood was made to stand at room temperature for 1 hour. It was then centrifuged and clear serum was separated. The serum was then stored for analysis. Reference: (Magari *et al.*, 2004).

**Serum biochemistry I** (ASPARTATE AMINOTRANSFERASE and ALANINE AMINOTRANSFERASE), Reference: Ritman S, Frankel S, (1957). A colorimeteric method for the determination of serum glutamic oxaloacetic and glutamic pyruvic transaminases. American Journal of Clinical Pathology; 28: 56-63.

**Procedure:** 0.2ml of serum, collected from test and control samples, was mixed with 1.0ml of buffer solution and incubated for 60 min at 37°C in a water bath. After the addition of 1.0ml of chromogen solution, the samples were kept at room temperature for 20minutes for the reaction to proceed and 10ml NaOH was added. The optical density was red at 546nm after 5min. To the blank, serum was added after the addition of chromogen solution.

**Serum Biochemistry II** (ALKALINE PHOSPHATASE), Reference: Klaus Walter and Schutt C. (1974). Acid and alkaline phosphatase in serum. In: Methods of Enzymatic Analysis.Eds. Hans Ulrich Bergmeyer, Verlag Chemie-

Weinheim,Academic press, Inc. New York, 2nd Edition, Vol.2, pp. 855-64.

**Procedure:** Alkaline phosphatase activity measurement is based on the ability of the enzyme to hydrolyze *p*-nitrophenol phosphate under alkaline conditions. The cleaved product p-nitrophenol is yellow in alkaline solution and is measured at 400-420 nm (Klaus and Schutt, 1974). 2.0 ml of buffered substrate was taken in the tubes 'Test' 'Control' and 'Blank' followed by addition of serum and distilled water (0.1ml) in 'Test' and 'Blank' respectively and incubated in a water bath for 30 minutes at 25°C. After incubation sodium hydroxide was added (0.25 N, 2 ml) to all the tubes, which was followed by serum (0.1ml) to the tubes marked 'Control'. The yellow colour formed was measured spectrophotometrically against blank at 410nm.

**Serum Biochemistry III** [BILIRUBIN (BRBN)], Reference: Malloy H T and Evelyn K A (1937). The determination of bilirubin with photoelectric colorimeter. Journal of Biological Chemistry 119: 481-490.

**Procedure:** Serum bilirubin was estimated by the method of Malloy and Evelyn (1937). In the two sets of test tubes marked 'Test' and 'Control', serum (0.2 ml) and distilled water (1.8 ml) were added. To the tubes marked 'Test' and 'Standard' diazo reagent (0.5 ml) was added. To the test tubes marked 'Control' and 'Blank' diazo blank (0.5 ml) was added. Finally methanol (2.5 ml) was added into each test tube. The tubes were mixed and allowed to stand for 30 minutes in dark. The tubes were read after 10 minutes at 540nm.

**Results:** Garcinol reduced increased levels of bilirubin and serum enzymes Alanine Transaminase (ALT), Aspartate Aminotransferase (AST) and Alkaline Phosphatase (ALP) in carbon tetrachloride induced liver damage in rats (Table 3).

**Table 3: Effect of Garcinol on bilirubin, ALT, AST and ALP in carbon tetrachloride induced liver damage in rats**

| SERUM PARAMETERS | | | | | | |
|---|---|---|---|---|---|---|
| Treatments | Dose (mg/kg) | ALT $\mu$mol/min/lt | AST $\mu$mol/min/lt | ALP $\mu$mol/min/lt | Bilirubin mg% | Average Protection % |
| Vehicle | - | 110.30±13.70 | 107.70±14.00 | 30.90±0.72 | 0.40±0.05 | - |
| Vehicle + CCl$_4$ | - | 1460.03±78.30 | 878.10±41.52 | 87.99±3.35 | 1.17±0.07 | - |
| GC+CCl$_4$ | 1.25 | 1272.50±109.19(12.84) | 746.81±36.93 (14.95) | 78.49±6.58 (10.79) | 1.03±0.04 (11.96) | 12.63 |
| GC+CCl$_4$ | 2.50 | 1027.07±68.26* (29.65) | 646.90±50.53* (26.32) | 70.32±3.89* (20.08) | 0.89±0.05** (23.93) | 24.99 |
| GC+CCl$_4$ | 5.00 | 828.59±46.56** (43.24) | 500.66±49.22** (42.98) | 59.19±4.45** (32.73) | 0.78±0.04** (33.33) | 38.07 |
| GC+CCl$_4$ | 10.00 | 891.36±45.72** (38.94) | 522.43±57.50** (40.50) | 59.70±3.69** (32.15) | 0.75±0.04** (35.89) | 36.87 |
| Silymarin + CCl$_4$ | 50 | 744.76±40.16** (48.99) | 460.76±25.60** (47.52) | 49.84±2.80** (43.35) | 0.64±0.04** (48.71) | 47.14 |
| (Values as Mean ± SE, n = 6) ; Percent change in parenthesis; P value * : < 0.01; ** : <0.001. (ALT) Alanine transaminase ; (AST) Aspartate aminotransferase ; (ALP) Alkaline phosphatase. | | | | | | |

## EXAMPLE 5

**Effect of different doses of Garcinol (GC) on TGF-beta 1 and ICAM-1 expression in liver homogenate of Paracetamol induced liver damage in rats.**

Animals used in the experiment: Male Wistar Rats

Weight of the animals: 140-160 grams

Doses of Garcinol (GC) used for the study: 1.25, 2.5, 5, 10 mg/kg p.o.

Standard drug: Silymarin (50 mg/kg) p. o.

Procedure: Experimental animals orally received paracetamol (400 mg/kg body weight) for seven days. The animals from drug treated group received 400 mg/kg body weight of paracetamol dissolved in water orally along with graded doses of test drug Garcinol for seven days. The standard group animals received 50 mg/kg body weight of standard drug silymarin and 400 mg/kg body weight of paracetamol for seven days and served as standard control. [N.Kanchana and A.Mohamed Sadiq, Hepatoprotective effect of Plumbago zeylanica on paracetamol induced liver toxicity in rats, Int J Pharm Pharm Sci, Vol 3, Issue1, 151-154 (2011*)*].

Liver tissue from experimental animals was homogenized on ice with a polytron and homogenate was centrifuged at 5000 g for 15 min. Aliquots of the supernatant were separated and used for biochemical analysis. Supernatants were stored at -80 °C until cytokine analysis. TGFβ and ICAM-1were estimated using commercially available kits based on sandwich and competitive ELISA technique according to the manufacturers' instructions. All cytokine concentrations were carried out by means of colorimetric measurement at 450 nm on an ELISA plate reader by interpolation from a standard curve. [(i) Magari K, Miyata S, Ohkubo Y, Mutoh S, (2004). Inflammatory cytokine levels in paw tissues during development of rat collagen-induced arthritis: Effect of FK506, an inhibitor of T cell activation. Inflammation Research. 53: 469-474 (Magari *et al.*, 2004); and (ii) Modulation of Th1/Th2 cytokines and inflammatory mediators by hydroxychavicol in adjuvant induced arthritic tissues. Anjali Pandey, Sarang Bani, Prabhu Dutt, Krishna Avtar Suri. Cytokine 49 (2010) 114-121].

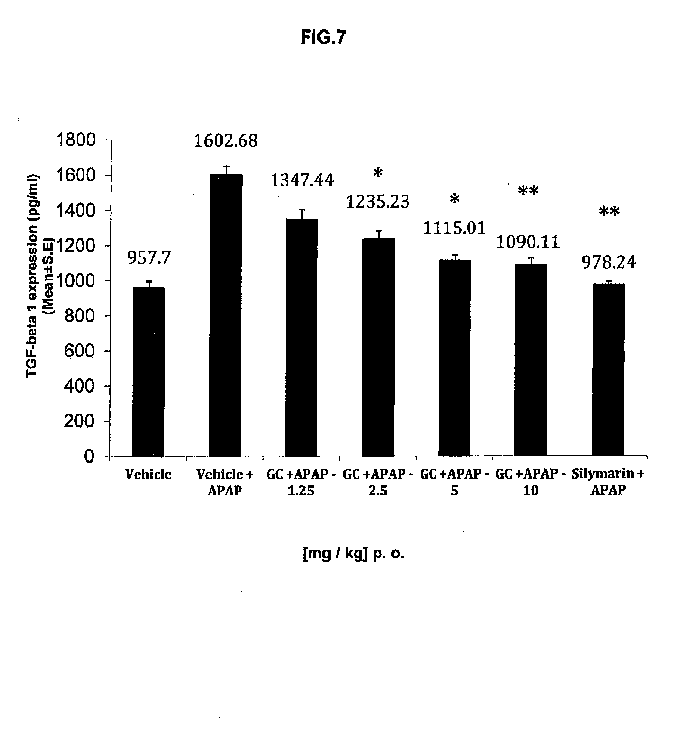

**Results:** Garcinol caused a dose dependent inhibition of increased levels of TGF-beta and ICAM-1 expressions in the liver associated with paracetamol induced acute hepatitis (FIG 7 and FIG 8).

## **EXAMPLE 6**

**Effect of different doses of Garcinol (GC) on serum enzymes Alanine Transaminase (ALT), Aspartate Aminotransferase (AST) and Alkaline Phosphatase (ALP) in paracetamol (acetyl-para-aminophenol-APAP) induced liver damage in rats**

Animals used in the experiment: Male Wistar Rats

Weight of the animals: 140-160 grams

Doses of Garcinol (GC) used for the study: 1.25, 2.5, 5, 10 mg/kg p.o.

Standard drug: Silymarin (50 mg/kg) p.o.

**Procedure:** Experimental animals orally received paracetamol (400 mg/kg body weight) for seven days. The animals from drug treated group received 400 mg/kg body weight ofparacetamol dissolved in water orally along with graded doses of test drug Garcinol for seven days. The standard group animals received 50 mg/kg body weight of standard drug silymarin and 400 mg/kg body weight of paracetamol for seven days and served as standard control. [N.Kanchana and A.Mohamed Sadiq, Hepatoprotective effect of Plumbago zeylanica on paracetamol induced liver toxicity in rats, Int J Pharm Pharm Sci, Vol 3, Issue1, 151-154 (2011*)*].

Blood was collected from the retro-orbital plexus of experimental animals and no anti-coagulant was added. Blood was made to stand at room temperature for 1 hour. It was then centrifuged and clear serum was separated. The serum was then stored for analysis. Reference: (Magari *et al.,* 2004).

**Serum biochemistry I** (ASPARTATE AMINOTRANSFERASE and ALANINE AMINOTRANSFERASE), Reference: Ritman S, Frankel S, (1957). A colorimeteric method for the determination of serum glutamic oxaloacetic and glutamic pyruvic transaminases. American Journal of Clinical Pathology; 28: 56-63.

**Procedure:** 0.2ml of serum, collected from test and control samples, was mixed with 1.0ml of buffer solution and incubated for 60 min at 37°C in a water bath. After the addition of 1.0ml of chromogen solution, the samples were kept at room temperature for 20 minutes for the reaction to proceed and 10ml NaOH was added. The optical density was red at 546nm after 5min. To the blank, serum was added after the addition of chromogen solution.

**Serum Biochemistry II** (ALKALINE PHOSPHATASE), Reference: Klaus Walter and Schutt C. (1974). Acid and alkaline phosphatase in serum. In: Methods of Enzymatic Analysis.Eds. Hans Ulrich Bergmeyer, Verlag Chemie-Weinheim,Academic press, Inc. New York, 2nd Edition, Vol.2, pp. 855-64.

**Procedure:** Alkaline phosphatase activity measurement is based on the ability of the enzyme to hydrolyze p-nitrophenol phosphate under alkaline conditions. The cleaved product *p*-nitrophenol is yellow in alkaline solution and is measured at 400-420 run (Klaus and Schutt, 1974). 2.0 ml of buffered substrate was taken in the tubes 'Test' 'Control' and 'Blank' followed by addition of serum and distilled water (0.1ml) in 'Test' and 'Blank' respectively and incubated in a water bath for 30 minutes at 25°C. After incubation sodium hydroxide was added (0.25 N, 2 ml) to all the tubes, which was followed by serum (0.1ml) to the tubes marked 'Control'. The yellow colour formed was measured spectrophotometrically against blank at 410nm.

**Serum Biochemistry III** [BILIRUBIN (BRBN)], Reference: Malloy H T and Evelyn K A (1937). The determination of bilirubin with photoelectric colorimeter. Journal of Biological Chemistry 119: 481-490.

**Procedure:** Serum bilirubin was estimated by the method of Malloy and Evelyn (1937). In the two sets of test tubes marked 'Test' and 'Control', serum (0.2 ml) and distilled water (1.8 ml) were added. To the tubes marked 'Test' and 'Standard' diazo reagent (0.5 ml) was added. To the test tubes marked 'Control' and 'Blank' diazo blank (0.5 ml) was added. Finally methanol (2.5 ml) was added into each test tube. The tubes were mixed and allowed to stand for 30 minutes in dark. The tubes were read after 10 minutes at 540nm.

**Results:** Garcinol reduced increased levels of bilirubin and serum enzymes Alanine Transaminase (ALT), Aspartate Aminotransferase (AST) and Alkaline Phosphatase (ALP) in paracetamol induced liver damage in rats (Table 4).

**Table 4: Effect of Garcinol on bilirubin, ALT, AST and ALP in paracetamol induced liver damage in rats**

| SERUM PARAMETERS | | | | | |
|---|---|---|---|---|---|
| Treatments | Dose (mg/kg) | ALT ($\mu$mol min/lt) | AST($\mu$mol min/lt) | ALP($\mu$mol) min/lt) | Average Protection% |
| Vehicle | - | 98.32±5.20 | 117.49±8.26 | 21.42±2.04 | - |
| Vehicle+APAP | - | 1102.10+46.98 | 839.79±59.17 | 40.54±3.61 | - |
| GC+APAP | 1.25 | 862.97±53.38 (21.69) | 761.61±43.96 (9.30) | 37.21±3.44 (8.21) | 13.06 |
| GC+APAP | 2.50 | 808.64±38.55* (26.62) | 650.88±35.84* (22.49) | 34.28±1.98 (15.44) | 21.51 |
| GC+APAP | 5.00 | 661.67±51.40** (39.96) | 463.41±38.56** (44.81) | 30.74±2.05** (24.17) | 36.31 |
| GC+APAP | 10.00 | 590.22±50.98** (46.44) | 536.39±44.13** (36.12) | 33.60±1.93* (17.11) | 33.22 |
| Silymarin + APAP | 50 | 377.04±14.44** (65.78) | 379.64±23.22** (54.79) | 26.90±2.24** (33.64) | 51.40 |
| **(Values as Mean ± SE, n = 6); Percent change in parenthesis; P value *: < 0.01; ** : <0.001. (ALT) Alanine transaminase ; (AST) Aspartate aminotransferase ; (ALP) Alkaline phosphatase** | | | | | |

## EXAMPLE 7

**Effect of different doses of Garcinol (GC) on TNF-$\alpha$ in the blood of paracetamol induced liver damage in rats.**

Animals used in the experiment: Male Wistar Rats

Weight of the animals: 140-160 grams

Doses of Garcinol (GC) used for the study: 1.25, 2.5, 5, 10 mg/kg p.o.

Standard drug: Silymarin (50 mg/kg) p.o.

**Procedure:** Experimental animals orally received paracetamol (400 mg/kg body weight) for seven days. The animals from drug treated group received 400 mg/kg body weight of paracetamol dissolved in water orally along with graded doses of test drug Garcinol for seven days. The standard group animals received 50 mg/kg body weight of standard drug silymarin and 400 mg/kg body weight of paracetamol for seven days and served as standard control. [N.Kanchana and A.Mohamed Sadiq, Hepatoprotective effect of Plumbago zeylanica on paracetamol induced liver toxicity in rats, Int J Pharm Pharm Sci, Vol 3, Issuel, 151-154 (2011)].

The animals were bled retro-orbitally and blood was collected in EDTA-coated tubes for the estimation of PE-labeled anti-rat TNF-alpha monoclonal antibody expression. Analysis was done on flow cytometer (BD-FACS CANTO II). These fluorochrome-labeled monoclonal antibodies were added directly to 100 $\mu$l of whole blood, which was then lysed using whole blood lysing reagent. Following the final centrifugation, samples were resuspended in phosphate-buffered saline (pH, 7.4) and analyzed directly on the flow cytometer. A fluorescence trigger was set on the PE (FL2) parameter to collect the events. [(i) Bani S, Kaul A, Khan B, Ahmad SF, Suri KA, Gupta BD, Satti NK, Qazi GN, (2006).Suppression of T lymphocyte activity by lupeol isolated from Crataeva religiosa. Phytotherapy Research; 20(4): 279-287. And (ii) Bani S, Kaul A, Khan B, Ahmad SF, Suri KA, Satti NK, (2005). Immunosuppressive properties of an ethyl acetate fraction from Euphorbia royleana. Journal of Ethnopharmacology; 99: 185-192].

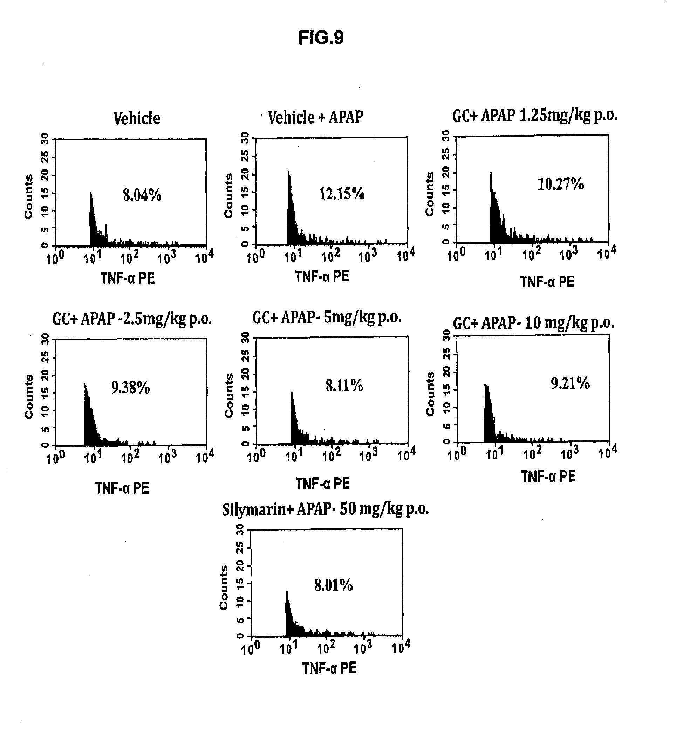

**Results: FIG 9** shows that Garcinol causes dose dependant reduction of increased levels of TNF-$\alpha$ in the liver occurring in acute hepatitis caused by drug (paracetamol) induced liver damage.

## EXAMPLE 8

**The effect of different doses of Garcinol (GC) on Tumor Necrosis Factor-$\alpha$ (TNF-$\alpha$), Interleukin-1$\beta$ (IL-1$\beta$) and IL-12 in the serum of alcohol (Ethyl alcohol-EtOH) induced hepatotoxic animal models.**

Animals used in the experiment: Male Wistar Rats

Weight of the animals: 140-160 grams

Doses of Garcinol (GC) used for the study: 1.25, 2.5, 5, 10 mg/kg p.o.

Standard drug: Silymarin (50 mg/kg) p.o.

Procedure: Male Wistar rats, weighing 180-200g, were given 0.5-0.6ml ethanol orally. The initial dose of ethanol was 6g/kg/day (solutions maximally containing 56% alcohol), and the dose was progressively increased during week 1 to a maintenance dose of 8 g/kg/day that was continued for 5 more weeks. All rats had regular standard rat chow available throughout the 6-week period. Rats were weighted three times per week. [Guangjin Yuan, Zuojiong Gong *, Xiaorong Zhou, Pin Zhang, Xiaomei Sun and Xi Li. Epigallocatechin-3-Gallate Ameliorates Alcohol-Induced Liver Injury in Rats. Int. J. Mol. Sci. 2006, 7, 204-219].

Blood was collected from the retro-orbital plexus of the alcohol treated experimental animals and mixed with EDTA for cytokine estimations. For collecting serum no anti-coagulant was added to the blood and it was made to stand at room temperature for 1h. The blood was then centrifuged and clear serum was separated and stored for analysis. TNF-$\alpha$, IL-1$\beta$ and IL-12 were estimated using commercially available kits based on sandwich and competitive ELISA technique according to the manufacturers' instructions. All cytokine concentrations were carried out by means of colorimetric measurement at 450 nm on an ELISA plate reader by interpolation from a standard curve.

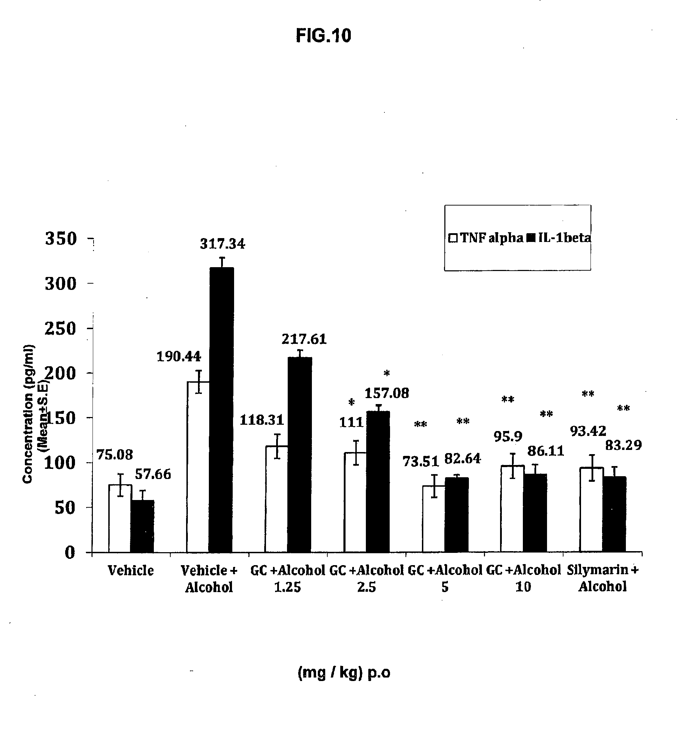

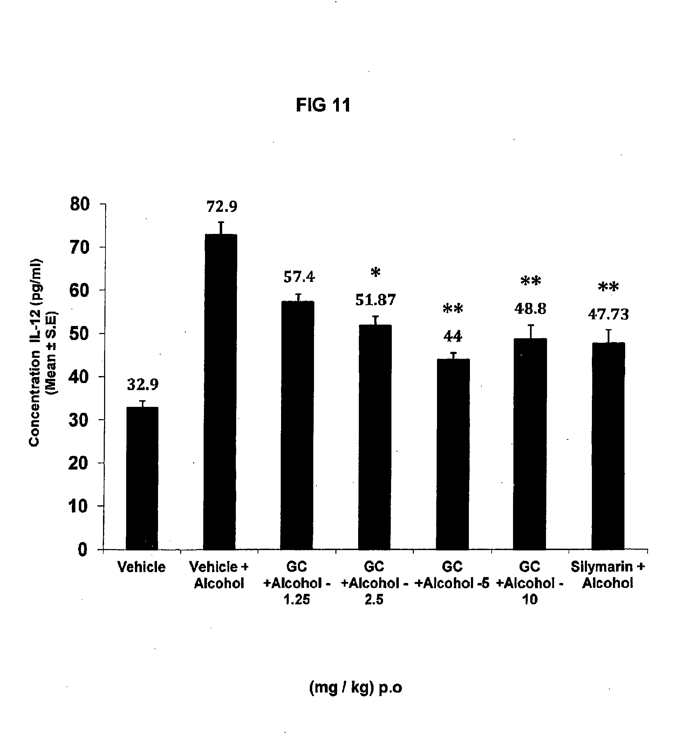

**Result:** Garcinol (GC) inhibited increased levels of TNF-alpha, Interleukin- 1 beta and Interleukin-12 (FIG 10 and FIG 11) induced by TLR-4 activation of Kupffer cells by LPS of gram negative bacteria in the gut the activation and excessive growth of which is due to ethyl alcohol intake.

## EXAMPLE 9

**Effect of different doses of Garcinol (GC) on serum enzymes Alanine Transaminase (ALT), Aspartate Aminotransferase (AST) and Alkaline Phosphatase (ALP) in in the serum of alcohol (Ethyl alcohol) induced hepatotoxic animal models**

Animals used in the experiment: Male Wistar Rats

Weight of the animals: 140-160 grams

Doses of Garcinol (GC) used for the study: 1.25, 2.5, 5, 10 mg/kg p.o.

Standard drug: Silymarin (50 mg/kg) p.o.

**Procedure:** Male Wistar rats, weighing 180-200g, were given 0.5-0.6ml ethanol orally. The initial dose of ethanol was 6g/kg/day (solutions maximally containing 56% alcohol), and the dose was progressively increased during week 1 to a maintenance dose of 8 g/kg/day that was continued for 5 more weeks. All rats had regular standard rat chow available throughout the 6-week period. Rats were weighted three times per week. [Guangjin Yuan, Zuojiong Gong *, Xiaorong Zhou, Pin Zhang, Xiaomei Sun and Xi Li. Epigallocatechin-3-Gallate Ameliorates Alcohol-Induced Liver Injury in Rats. Int. J. Mol. Sci. 2006, 7, 204-219].Blood was collected from the retro-orbital plexus of experimental animals and no anti-coagulant was added. Blood was made to stand at room temperature for 1 hour. It was then centrifuged and clear serum was separated. The serum was then stored for analysis. Reference: (Magari *et al.,* 2004).

**Serum biochemistry I** (ASPARTATE AMINOTRANSFERASE and ALANINA AMINOTRANSFERASE), Reference: Ritman S, Frankel S, (1957). A colorimetric method for the determination of serum glutamic oxaloacetic and glutamic pyruvic transaminases. American Journal of Clinical Pathology; 28: 56-63.

**Procedure:** 0.2ml of serum, collected from test and control samples, was mixed with 1.0ml of buffer solution and incubated for 60 min at 37°C in a water bath. After the addition of 1.0ml of chromogen solution, the samples were kept at room temperature for 20minutes for the reaction to proceed and 10ml NaOH was added. The optical density was red at 546nm after 5min. To the blank, serum was added after the addition of chromogen solution.

**Serum Biochemistry II** (ALKALINE PHOSPHATASE), Reference: Klaus Walter and Schutt C. (1974). Acid and alkaline phosphatase in serum. In: Methods of Enzymatic Analysis.Eds. Hans Ulrich Bergmeyer, Verlag Chemie-Weinheim,Academic press, Inc. New York, 2nd Edition, Vol.2, pp. 855-64.

**Procedure:** Alkaline phosphatase activity measurement is based on the ability of the enzyme to hydrolyze p-nitrophenol phosphate under alkaline conditions. The cleaved product p-nitrophenol is yellow in alkaline solution and is measured at 400-420 nm (Klaus and Schutt, 1974). 2.0 ml of buffered substrate was taken in the tubes 'Test' 'Control' and 'Blank' followed by addition of serum and distilled water (0.1ml) in 'Test' and 'Blank' respectively and incubated in a water bath for 30 minutes at 25°C. After incubation sodium hydroxide was added (0.25 N, 2 ml) to all the tubes, which was followed by serum (0.1ml) to the tubes marked 'Control'. The yellow colour formed was measured spectrophotometrically against blank at 410nm.

**Serum Biochemistry III** [BILIRUBIN (BRBN)], Reference: Malloy H T and Evelyn K A (1937). The determination of bilirubin with photoelectric colorimeter. Journal of Biological Chemistry 119: 481-490.

**Procedure:** Serum bilirubin was estimated by the method of Malloy and Evelyn (1937). In the two sets of test tubes marked 'Test' and 'Control', serum (0.2 ml) and distilled water (1.8 ml) were added. To the tubes marked 'Test' and 'Standard' diazo reagent (0.5 ml) was added. To the test tubes marked 'Control' and 'Blank' diazo blank (0.5 ml) was added. Finally methanol (2.5 ml) was added into each test tube. The tubes were mixed and allowed to stand for 30 minutes in dark. The tubes were read after 10 minutes at 540nm.

**Table 5**

| SERUM PARAMETERS | | | | | | |
|---|---|---|---|---|---|---|
| Treatments | Dose (mg/kg) | ALT ($\mu$mol min/lt) | AST($\mu$mol min/lt) | ALP($\mu$mol min/lt) | Bilirubin mg% | Average Protection% |
| Vehicle | - | 118.10±10.20 | 106.40±16.60 | 32.30±0.52 | 0.44±0.08 | - |
| Vehicle+ EtOH | - | 1820.08±42.80 | 1076.32 ±48.22 | 104.22±8.60 | 2.08±0.02 | - |
| GC + EtOH | 1.25 | 1320.40±52.20* (27.45) | 858.80±32.64 (20.20) | 88.68 ±8.58 (14.91) | 1.94±0.02 (6.73) | 17.32 |
| GC + EtOH | 2.50 | 1080.50±48.56** (40.63) | 726.78±46.50* (32.47) | 76.12±4.88* (26.96) | 1.68±0.04* (19.23) | 29.82 |
| GC + EtOH | 5.00 | 876.58±76.80** (51.83) | 650.26±56.20** (39.58) | 68.32±5.60** (34.44) | 1.42±0.06** (31.73) | 39.39 |
| GC + EtOH | 10.00 | 870.80±48.40** (52.15) | 632.48±44.56** (41.23) | 69.00±4.82** (33.79) | 1.39±0.04** (33.65) | 40.20 |
| Silymarin + EtOH | 50 | 854.88±38.46** (53.03) | 662.70±38.40** (38.44) | 66.54±6.30** (36.15) | 1.34±0.02** (35.57) | 40.79 |
| (Values as Mean ± SE, n = 6) ; Percent change in parenthesis; P value * : < 0.01; ** : <0.001. (ALT) Alanine transaminase ; (AST) Aspartate aminotransferase ; (ALP) Alkaline phosphatase | | | | | | |

**Result:** Garcinol (GC) reduced the increased levels of AST, ALT, ALP and Bilirubin in ethyl alcohol induced hepatotoxic experimental models.

**EXAMPLE 10**

**The cytotoxic potential (% cytotoxicity) of garcinol in Hep G2 liver cancer cell line in comparison with Silymarin, a known hepatoprotective agent (Overall hepatoprotective effect of Garcinol vs Silymarin-Tables 6 and 7, FIG 12 and FIG 13)**

Procedure- HEP G2 cells grown in varying concentrations of the material to be checked for cytotoxicity are taken. The medium is then tapped off gently and 100 ml of working stock solution of MTT (150 mg/well) is added into each well. Then the plate is further wrapped in aluminium foil and incubated for 4 hours in $CO_2$ incubator at 37°C. The plate is then washed gently with 100 ml of PBS per well. The washing must be done soon after tapping off the medium to avoid drying, flaking and loss of cells during washing. Solubilize the dye in 100 ml of DMSO per well. The plates are shaken for 5 minutes and absorbance read at 492 nm using a Fluostar optima (BMG) micro plate reader. The absorbance will be directly proportional to the cell viability. The option of reading at 620nm also can be adopted which would deduct the interference of the cell debris in the samples. The data is analyzed by plotting cell number versus absorbance allowing the quantification of changes in cell proliferation. The rate of tetrazolium reduction is proportional to the rate of cell proliferation.

**Calculation:**

Cytotoxicity of the sample is expressed as $IC_{50}$ value, the concentration which inhibits 50% of the cell growth.

$$\% \text{ Cytotoxicity} = \frac{E - T}{E} \times 100$$

Where, E = Cell viability in the absence of the sample
T = Cell viability in the presence of the sample.
Hepatoprotective activity of Garcinol in HEP G2 cells
$IC_{50}$ = 13.33ug/ml
95%CL = 11.84 to 15.02ug/ml
Hepatoprotective activity of Silymarin in HEP G2 cells
IC 50 = 36.51ug/ml
95%CL = 32.18 to 41.42ug/ml
Lesser the $IC_{50}$ value, better the efficacy.

**Table 6:** [Cytotoxicity of Silymarin in Hep G2 cell line]

| Concentration (μg/ml) | % Cytotoxicity |
|---|---|
| 100.00 | 81.77 |
| 50.00 | 64.33 |
| 25.00 | 8.62 |
| 12.50 | -7.46 |
| 6.25 | -4.25 |
| 3.13 | -11.27 |
| 1.56 | -8.63 |
| 0.78 | -8.31 |
| 0.39 | 2.34 |
| Sigmoidal Dose Response (variable slope) | |
| BOTTOM | 0 |
| TOP | 100 |
| $IC_{50}$ | 36.51 μg/ml |
| 95% CL | 32.18 to 41.42 μg/ml |
| $R^2$ | 0.9545 |

**Table 7:** [Cytotoxicity of Garcinol in Hep G2 cell line]

| Concentration (μg/ml) | % Cytotoxicity |
|---|---|
| 200.00 | 79.50 |
| 100.00 | 83.44 |
| 50.00 | 83.15 |
| 25.00 | 60.90 |
| 12.50 | 35.81 |
| 6.25 | -1.94 |
| 3.13 | -14.65 |
| 1.56 | -12.09 |
| 0.78 | -3.81 |

(continued)

| Sigmoidal Dose Response (variable slope) | |
| --- | --- |
| BOTTOM | 0 |
| TOP | 100 |
| $IC_{50}$ | 13.33 $\mu$g/ml |
| 95% CL | 11.84 to 15.02 $\mu$g/ml |
| $R^2$ | 0.9756 |

**Results:** Garcinol shows comparative hepatoprotective effect like Silymarin at lower concentrations (values below 6.25 $\mu$g/ml).

While the invention has been described with reference to a preferred embodiment, it is to be clearly understood by those skilled in the art that the invention is not limited thereto. Rather, the scope of the invention is to be interpreted only in conjunction with the appended claims.

**Claims**

1.  A method of mammalian hepatocyte protection, said method comprising the step of bringing into contact mammalian hepatocytes with an effective concentration of garcinol, wherein any method practiced as a method for treatment of the human or animal body by therapy or practiced as a diagnostic method on the human or animal body is excluded.

2.  The method according to claim 1, wherein the effective concentration of garcinol is from about 0.78 $\mu$g/ml to about 6.25 $\mu$g/ml.

3.  A method of reducing increased levels of cytokine expression in mammalian models of liver damage (hepatotoxicity), said method comprising the step of administering an effective amount of garcinol to said models.

4.  The method according to claim 3, wherein the cytokine is Transforming Growth Factor G-$\beta$1 (TGF-$\beta$1), Tumor Necrosis Factor-$\alpha$, Interleukin-2 (IL-2), Interleukin-4 (IL-4), or Interleukin-12 (IL-12).

5.  The method according to claim 3, wherein the hepatotoxicity is caused by toxin, by drugs or by ethyl alcohol.

6.  A method of reducing increased levels of adhesion molecule expression in mammalian models of liver damage (hepatotoxicity), said method comprising the step of administering an effective amount of garcinol to said models.

7.  The method according to claim 6, wherein the adhesion molecule is Intracellular Adhesion Molecule-1 (ICAM-1 or CD 52).

8.  The method according to claim 6, wherein the hepatotoxicity is caused by toxin.

9.  The method according to claim 6, wherein the hepatotoxicity is caused by drugs.

10. A method of reducing elevated levels of liver enzymes and/or bile pigments in mammalian models of liver damage (hepatotoxicity), said method comprising the step of administering an effective amount of garcinol to said models.

11. The method according to claim 10, wherein the hepatotoxicity is caused by toxin.

12. The method according to claim 10, wherein the hepatotoxicity is caused by drugs or by ethyl alcohol.

13. The method according to claim 10, wherein the liver enzyme is selected from a group comprising Alanine Transaminase, Aspartate aminotransferase and Alkaline Phosphatase.

14. The method according to claim 10, wherein the bile pigment is bilirubin.

**15.** Garcinol for use as a medicament for providing hepatoprotection.

**Patentansprüche**

**1.** Verfahren zum Schutz von Säugetier-Hepatozyten, wobei das Verfahren den Schritt des Inkontaktbringens von Säugetier-Hepatozyten mit einer wirksamen Konzentration von Garcinol umfasst, wobei jedes Verfahren, welches als ein Verfahren zur therapeutischen Behandlung des menschlichen oder tierischen Körpers verübt wird oder als ein diagnostisches Verfahren am menschlichen oder tierischen Körper verübt wird, ausgeschlossen ist.

**2.** Verfahren nach Anspruch 1, wobei die wirksame Konzentration von Garcinol von etwa 0,78 $\mu$g/ml bis etwa 6,25 $\mu$g/ml beträgt.

**3.** Verfahren zum Reduzieren gesteigerter Pegel von Zytokin-Expression in Säugetiermodellen der Leberschädigung (Hepatotoxizität), wobei das Verfahren den Schritt des Verabreichens einer wirksamen Menge von Garcinol an diese Modelle umfasst.

**4.** Verfahren nach Anspruch 3, wobei das Zytokin Transformierender Wachstumsfaktor G-$\beta$1 (TGF-$\beta$1), Tumornekrosefaktor-$\alpha$, Interleukin-2 (IL-2), Interleukin-4 (IL-4) oder Interleukin-12 (IL-12) ist.

**5.** Verfahren nach Anspruch 3, wobei die Hepatotoxizität durch Toxin, durch Medikamente oder durch Ethylalkohol verursacht ist.

**6.** Verfahren zum Reduzieren gesteigerter Pegel von Adhäsionsmolekül-Expression in Säugetiermodellen der Leberschädigung (Hepatotoxizität), wobei das Verfahren den Schritt des Verabreichens einer wirksamen Menge von Garcinol an diese Modelle umfasst.

**7.** Verfahren nach Anspruch 6, wobei das Adhäsionsmolekül Intrazelluläres Adhäsionsmolekül-1 (ICAM-1 oder CD 52) ist.

**8.** Verfahren nach Anspruch 6, wobei die Hepatotoxizität durch Toxin verursacht ist.

**9.** Verfahren nach Anspruch 6, wobei die Hepatotoxizität durch Medikamente verursacht ist.

**10.** Verfahren zur Reduktion erhöhter Pegel von Leberenzymen und/oder Gallepigmenten in Säugetiermodellen der Leberschädigung (Hepatotoxizität), wobei das Verfahren den Schritt des Verabreichens einer wirksamen Menge von Garcinol an diese Modelle umfasst.

**11.** Verfahren nach Anspruch 10, wobei die Hepatotoxizität durch Toxin verursacht ist.

**12.** Verfahren nach Anspruch 10, wobei die Hepatotoxizität durch Medikamente oder durch Ethylalkohol verursacht ist.

**13.** Verfahren nach Anspruch 10, wobei das Leberenzym ausgewählt ist aus einer Gruppe umfassend Alanin-Transaminase, Aspartat-Aminotransferase und Alkalische Phosphatase.

**14.** Verfahren nach Anspruch 10, wobei das Gallepigment Bilirubin ist.

**15.** Garcinol zur Verwendung als ein Medikament zum Bereitstellen eines Leberschutzes.

**Revendications**

**1.** Procédé de protection d'hépatocytes de mammifères, ledit procédé comprenant l'étape consistant à amener en contact des hépatocytes de mammifères avec une concentration efficace en garcinol, dans lequel tout procédé pratiqué comme procédé de traitement du corps humain ou animal par thérapie ou pratiqué comme procédé diagnostique sur le corps humain ou animal est exclu.

**2.** Procédé selon la revendication 1, dans lequel la concentration efficace en garcinol va d'environ 0,78 $\mu$g/ml à environ

6,25 µg/ml.

3. Procédé de réduction de niveaux accrus d'expression de cytokines dans des modèles de mammifères de lésion hépatique (hépatotoxicité), ledit procédé comprenant l'étape consistant à administrer une quantité efficace de garcinol auxdits modèles.

4. Procédé selon la revendication 3, dans lequel la cytokine est le facteur de croissance transformant G-β1 (TGF-β1), le facteur de nécrose tumorale-α, l'interleukine-2 (IL-2), l'interleukine-4 (IL-4) ou l'interleukine-12 (IL-12).

5. Procédé selon la revendication 3, dans lequel l'hépatotoxicité est provoquée par une toxine, par des médicaments ou par de l'alcool éthylique.

6. Procédé de réduction de niveaux accrus d'expression de molécules d'adhésion dans des modèles de mammifères de lésion hépatique (hépatotoxicité), ledit procédé comprenant l'étape consistant à administrer une quantité efficace de garcinol auxdits modèles.

7. Procédé selon la revendication 6, dans lequel la molécule d'adhésion est la molécule d'adhésion intracellulaire-1 (ICAM-1 ou CD 52).

8. Procédé selon la revendication 6, dans lequel l'hépatotoxicité est provoquée par une toxine.

9. Procédé selon la revendication 6, dans lequel l'hépatotoxicité est provoquée par des médicaments.

10. Procédé de réduction de niveaux élevés d'enzymes hépatiques et/ou de pigments biliaires dans des modèles de mammifères de lésion hépatique (hépatotoxicité), ledit procédé comprenant l'étape consistant à administrer une quantité efficace de garcinol auxdits modèles.

11. Procédé selon la revendication 10, dans lequel l'hépatotoxicité est provoquée par une toxine.

12. Procédé selon la revendication 10, dans lequel l'hépatotoxicité est provoquée par des médicaments ou par de l'alcool éthylique.

13. Procédé selon la revendication 10, dans lequel l'enzyme hépatique est sélectionnée parmi un groupe comprenant l'alanine transaminase, l'aspartate aminotransférase et la phosphatase alcaline.

14. Procédé selon la revendication 10, dans lequel le pigment biliaire est la bilirubine.

15. Garcinol destiné à être utilisé comme médicament pour fournir une hépatoprotection.

## FIG.1

**FIG.2**

**FIG.3**

# FIG.4

FIG.5

Vehicle

Vehicle+CCl$_4$

GC+ CCl4 1.25mg/kg p.o.

8.03%

15.57%

13.03%

GC+ CCl4 2.5mg/kg p.o.

GC+ CCl4 5mg/kg p.o.

GC+ CCl4 10 mg/kg p.o.

12.18%

10.07%

10.17%

Silymarin+ CCl4 50 mg/kg p.o.

9.89%

FIG.6

Vehicle

Vehicle+CCl$_4$

GC+ CCl4 1.25mg/kg p.o.

GC+ CCl4 2.5mg/kg p.o.

GC+ CCl4 5mg/kg p.o.

GC+ CCl4 10 mg/kg p.o.

Silymarin+ CCl4 50 mg/kg p.o.

**FIG.7**

**FIG.8**

## FIG.9

FIG.10

Concentration (pg/ml) (Mean±S.E)

□ TNF alpha ■ IL-1beta

350
300
250
200
150
100
50
0

75.08   57.66   190.44   317.34   118.31   217.61   111   157.08   73.51   82.64   95.9   86.11   93.42   83.29

Vehicle | Vehicle + Alcohol | GC +Alcohol 1.25 | GC +Alcohol 2.5 | GC +Alcohol 5 | GC +Alcohol 10 | Silymarin + Alcohol

(mg / kg) p.o

FIG 11

FIG 12

Cytotoxicity of Silymarin in Hep
G2 cell line

Cytotoxicity of Garcinol in Hep G2 cell
line

**FIG.13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 61428643 A

**Non-patent literature cited in the description**

- **NILESH MEHTA et al.** *Drug-Induced Hepatotoxicity-Medscape REFERENCE-Drugs, Diseases and Procedures*
- **TANAKA, T.** Prevention of colonic aberrant crypt foci by dietary feeding of garcinol in male F3444 rats. *Carcinogenesis,* June 2000, vol. 21 (6), 1183-9
- **CHENG, A.-C. et al.** *Food Funct.,* 2010, vol. 1, 301-307
- **LINUMA M et al.** Antibacterial activity of some Garcinia benzophenone derivatives against methicillin-resistant Staphylococcus aureus. *Biol Pharm Bull,* February 1996, vol. 19 (2), 311-4
- **TAPAS et al.** Polyisoprenylated Benzophenone, Garcinol, a Natural Histone Acetyl transferase Inhibitor, Represses Chromatin Transcription and Alters Global GeneExpression. *The Journal of Biological Chemistry,* 06 August 2004, vol. 279 (32), 33716-33726
- OECD Guidelines for Testing of Chemicals. Guideline 423, Acute Oral Toxicity - Acute Toxic Class Method, Adopted. *Organization for Economic Cooperation and Development,* 1996
- **BRAMANTI G ; MURMANN W ; PIERINI P ; COMPORTI M.** Effect of cicloxilic acid on CCl4 - induced liver injury. *Drug Research,* 1978, vol. 28, 1112-1217
- **B SINGH ; A K SAXENA ; B K CHANDAN ; K K ANAND.** Hepatoprotective activity of Verbenalin on experimental liver damage in rodents. *Fitoterapia LXIX,* 1998, vol. 2, 135-140
- **B.K. CHANDAN ; A.K. SHARMA ; K.K. ANAND.** Boerhaavia diffusa: A study of its hepatoprotective activity. *Journal of Ethnopharmacology,* March 1991, vol. 31 (3), 299-307
- **MAGARI K ; MIYATA S ; OHKUBO Y ; MUTOH S.** Inflammatory cytokine levels in paw tissues during development of rat collagen-induced arthritis: Effect of FK506, an inhibitor of T cell activation. *Inflammation Research.,* 2004, vol. 53, 469-474
- **ANJALI PANDEY ; SARANG BANI ; PRABHU DUTT ; KRISHNA AVTAR SURI.** Modulation of Thl/Th2 cytokines and inflammatory mediators by hydroxychavicol in adjuvant induced arthritic tissues. *Cytokine,* 2010, vol. 49, 114-121

- **BANI S ; KAUL A ; KHAN B ; AHMAD SF ; SURI KA ; GUPTA BD ; SATTI NK ; QAZI GN.** Suppression of T lymphocyte activity by lupeol isolated from Crataeva religiosa. *Phytotherapy Research,* 2006, vol. 20 (4), 279-287
- **BANI S ; KAUL A ; KHAN B ; AHMAD SF ; SURI KA ; SATTI NK.** Immunosuppressive properties of an ethyl acetate fraction from Euphorbia royleana. *Journal of Ethnopharmacology,* 2005, vol. 99, 185-192
- **BRAMANTI G ; MURMANN W ; PIERINI P ; COMPORTI M.** Effect of cicloxilic acid on CCl4 - induced liver injury. *Drug Research,* 1978, vol. 28, 1112-1217
- **RITMAN S ; FRANKEL S.** A colorimeteric method for the determination of serum glutamic oxaloacetic and glutamic pyruvic transaminases. *American Journal of Clinical Pathology,* 1957, vol. 28, 56-63
- Acid and alkaline phosphatase in serum. **KLAUS WALTER ; SCHUTT C.** Methods of Enzymatic Analysis. Verlag ChemieWeinheim,Academic press, Inc, 1974, vol. 2, 855-64
- **MALLOY H T ; EVELYN K A.** The determination of bilirubin with photoelectric colorimeter. *Journal of Biological Chemistry,* 1937, vol. 119, 481-490
- **N.KANCHANA ; A.MOHAMED SADIQ.** Hepatoprotective effect of Plumbago zeylanica on paracetamol induced liver toxicity in rats. *Int J Pharm Pharm Sci,* 2011, vol. 3 (1), 151-154
- **ANJALI PANDEY ; SARANG BANI ; PRABHU DUTT ; KRISHNA AVTAR SURI.** Modulation of Th1/Th2 cytokines and inflammatory mediators by hydroxychavicol in adjuvant induced arthritic tissues. *Cytokine,* vol. 49, 114-121
- The determination of bilirubin with photoelectric colorimeter. **MALLOY H T ; EVELYN K A.** Journal of Biological Chemistry. 1937, vol. 119, 481-490
- **N.KANCHANA ; A.MOHAMED SADIQ.** Hepatoprotective effect of Plumbago zeylanica on paracetamol induced liver toxicity in rats. *Int J Pharm Pharm Sci,* 2011, vol. 3, 151-154

- **GUANGJIN YUAN ; ZUOJIONG GONG * ; XI-AORONG ZHOU ; PIN ZHANG ; XIAOMEI SUN ; XI LI.** Epigallocatechin-3-Gallate Ameliorates Alcohol-Induced Liver Injury in Rats. *Int. J. Mol. Sci.,* 2006, vol. 7, 204-219
- **GUANGJIN YUAN ; ZUOJIONG GONG ; XI-AORONG ZHOU ; PIN ZHANG ; XIAOMEI SUN ; XI LI.** Epigallocatechin-3-Gallate Ameliorates Alcohol-Induced Liver Injury in Rats. *Int. J. Mol. Sci.,* 2006, vol. 7, 204-219
- **RITMAN S ; FRANKEL S.** A colorimetric method for the determination of serum glutamic oxaloacetic and glutamic pyruvic transaminases. *American Journal of Clinical Pathology,* 1957, vol. 28, 56-63
- **MALLOY H T ; EVELYN K A.** The determination of bilirubin with photoelectric colorimeter. *Journal of Biological Chemistry,* 1937, vol. 119, 481-490